Europäisches Patentamt

European Patent Office

Office européen des brevets

(11) Publication number: **0 412 526 A2**

# EUROPEAN PATENT APPLICATION

(21) Application number: 90115226.4

(22) Date of filing: 08.08.90

(51) Int. Cl.5: **C12N 15/15, C12P 21/02, C12N 15/62, C07H 21/04, C12N 1/21, //C12N15/54, (C12N1/21,C12R1:19)**

(30) Priority: 10.08.89 JP 207200/89

(43) Date of publication of application:
**13.02.91 Bulletin 91/07**

(84) Designated Contracting States:
**BE DE DK ES FR GB GR IT LU NL**

(71) Applicant: **NIPPON MINING COMPANY LIMITED**
**10-1, Toranamon 2-chome**
**Minato-ku Tokyo(JP)**

(72) Inventor: **Misawa, Satoru, c/o Nippon Mining Co. Ltd.**
17-35, Niizominami 3-chome
Toda-shi, Saitama(JP)
Inventor: **Matsuda, Hitoshi, c/o Nippon Mining Co. Ltd.**
17-35, Niizominami 3-chome
Toda-shi, Saitama(JP)
Inventor: **Abe, Shinichiro, c/o Nippon Mining Co. Ltd.**
17-35, Niizominami 3-chome
Toda-shi, Saitama(JP)

(74) Representative: **Boeters, Hans Dietrich, Dr.**
**Boeters & Bauer Bereiteranger 15**
**D-8000 München 90(DE)**

(54) **Expression vector for hirudin, hirudin fusion protein, transformed microorganism, and method for production of hirudin.**

(57) Expression method for hirudin or its variant which is highly expressed and efficiently produced according to the preparation of vector which hirudin or its variant is expressed in the form of a fused protein with all or a part of porcine adenylate kinase, following transformation of Escherichia coli with prepared vector and culturing

Moreover, when methionine codon of porcine adenylate kinase is substituted by leucine codon, expression of hirudin and so on can be carried out more efficiently.

Fig. 1

**EP 0 412 526 A2**

# EXPRESSION VECTOR FOR HIRUDIN, HIRUDIN FUSION PROTEIN, TRANSFORMED MICROORGANISM, AND METHOD FOR PRODUCTION OF HIRUDIN.

## (2) BACKGROUND OF THE INVENTION

The present invention relates to an expression vector for hirudin or its variant, a hirudin fusion protein, a transformed microorganism which is made by introducing the vector into Escherichia coli , and a method for mass production of hirudin or its variant, using the transformed microorganism.

## (3) DESCRIPTION OF THE PRIOR ART

Hirudin is a peptide found in a medicinal leech, Hirudo medicinalis Linn . Since the peptide has an action to block the blood coagulation through the specific inhibition of thrombin activity, it is noteworthy to apply it for therapeutic use and the studies for its application has been carried out.

Recently, the amino acid sequence of this protein was confirmed [FEBS LETTERS Vol. 165, No.2, p180-184 (1984), Biol. Chem. Hoppe-Seyler Vol. 367, p803-811 (1986)]. It is shown that the protein consists of 65 amino acids sequence and tyrosine residue at position 63 is sulfated characteristically. It has been very difficult to provide a large quantity of hirudin for the therapeutic use because of limited availability of purified hirudin from leeches.

Therefore, several trials to produce hirudin by gene technology have been proposed [For example, PCT8504418 or EP158564, EP168342, EP171024], however, there has not been a satisfactory method yet.

Moreover, when hirudin is produced by the gene technology the characteristic sulfated tyrosine at position 63 is desulfated, that is, desulfated hirudin (this is called only "hirudin" in the following) is expressed. It has been clarified accordingly with the progress of the molecular biological studies that there are several variants of hirudin which have different amino acid sequences [ibd., Japanese Laid-Open No. 60-23 3098, US4791100 or EP209061, PCT8603493, EP207956 or US4767742, PCT8603517 or EP236330, EP193175, EP273800].

[Problem To Be Dissolved By The Invention]

The present invention is to provide the expression vector with which hirudin or its variant can be highly expressed and efficiently produced, and thereby to provide the method by which hirudin or its variant can be produced in large quantities and at a low cost using gene technology.

## (4) SUMMARY OF THE INVENTION

It is an object of the present invention to provide the new expression vector with which hirudin or its variant can be highly expressed and efficiently produced. The other object of the present invention is to provide a microorganism ( Escherichia coli ) which has been transformed by the said expression vector Moreover, the other object of the present invention is to provide the production method which said microorganism is cultured, fused protein of hirudin or its variant is expressed, and hirudin and its variant is purified. Further, the other object of the present invention is to provide new hirudin fusion protein.

These and other objects have been attained by the use of a new vector with which fusion protein consisting of porcine adenylate kinase and hirudin or a hirudin variant can be expressed .

Further, the present invention can be used a DNA coding for porcine adenylate kinase with which some amino acid residues are substituted by other amino acid residues.

Therefore, the present invention relates to

(1) An expression vecter for hirudin which comprises of DNA fragments containing tac or trp promotor region, replication origin of plasmid pUC18, all or a part of porcine adenylate kinase coding sequence, and hirudin or its variant coding sequence, and which is characterized to express a fused protein of hirudin or its variant with all or a part of porcine adenylate kinase.

(2) A hirudin fusion protein which comprises of the all or a part of porcine adenylate kinase and hirudin or its variant.

(3) A transformed microorganism which is made by introducing the vector according to the above (1) into Escherichia coli .

(4) A production method of hirudin which is characterized in that the transformed microorganism according to the above (3) are cultured, the hirudin fusion protein is expressed, and hirudin or its variant is recovered by cleavage of the fusion protein following its separation.

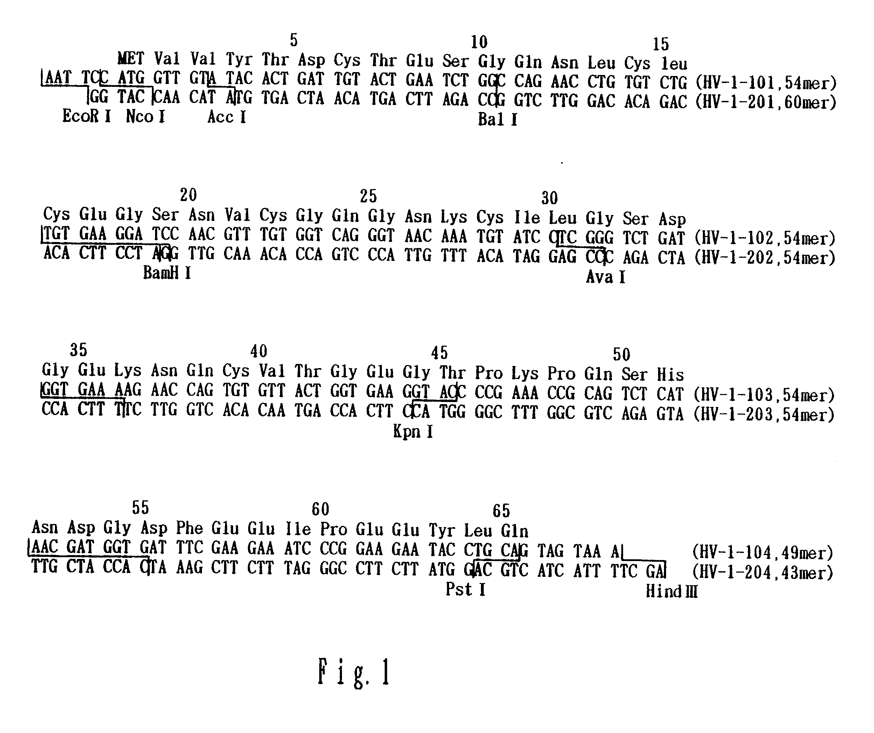

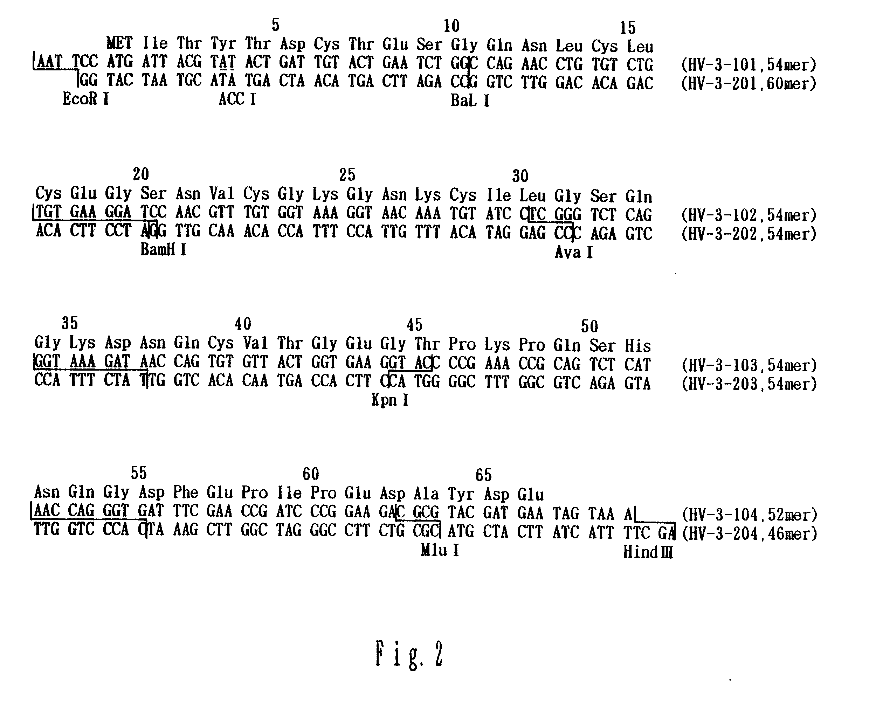

Hirudin HV-1, HV-2 and HV-3 are known as the above mentioned hirudin. Hirudin variants in which some amino acids are substituted by other amino acids or omitted are also known (reffered above). Although either hirudin or these hirudin variants can be applied in the present invention, HV-1 and HV-3 are preferentially suitable with regard to their pharmacological activities and so on. Their amino acid sequences and nucleotide sequences designed for them are shown in Fig.1 and Fig.2.

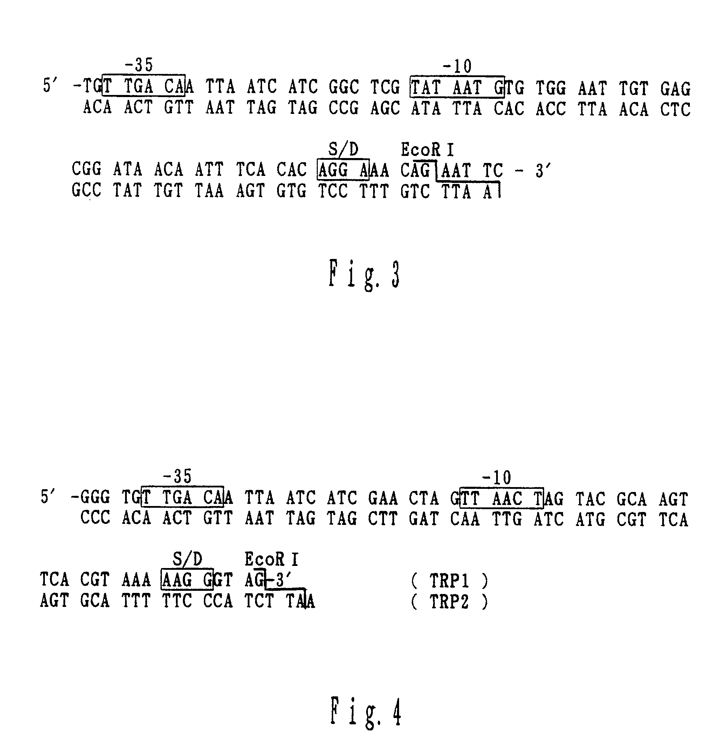

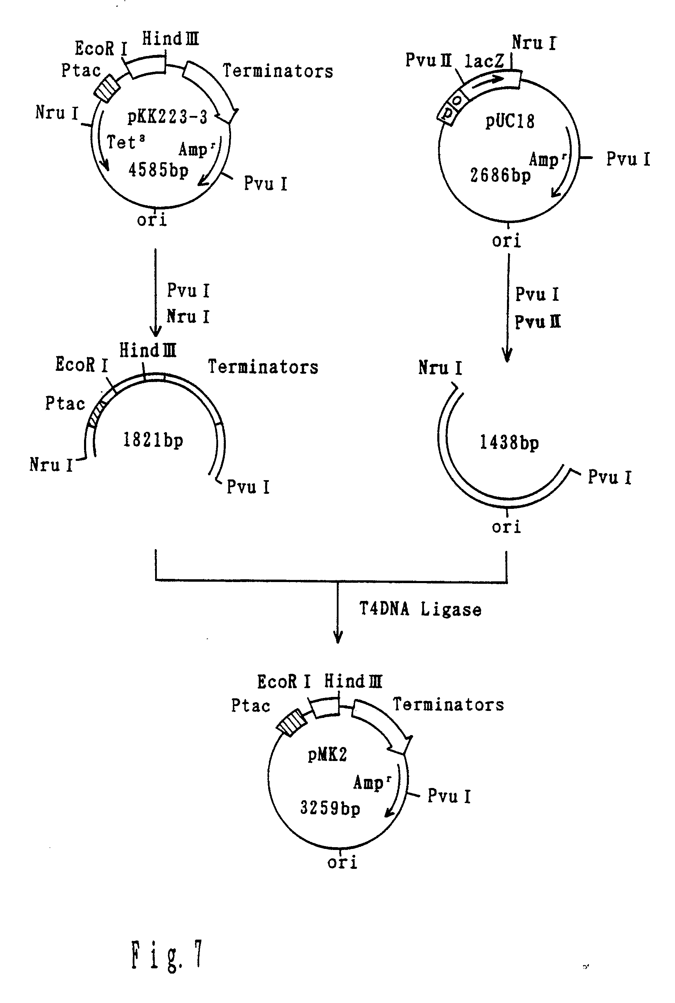

Tac promoter or trp promoter region mentioned in the present invention have their nucleotide sequences shown in Fig.3 and 4,respectively, and they can be easily synthesized by a DNA synthesizer and so on. Therefore, the synthesized promoter can be used for joining the replication origin of plasmid pUC18 to construct the vector in the present invention. However, commercially available plasmids can be used for the vector construction. For example, Pvul - Nrul fragment of plasmid pKK 223-3 ( Pharmacia ) including tac promoter region and Pvul - Nrul fragment of plasmid pUC18 including replication origin are ligated with T4 DNA ligase to yield plasmid pMK2 as shown in Fig. 7. And then all or a part of cDNA of porcine adenylate kinase is inserted into the plasmid. Thus, a vector which comprises the DNA fragments containing tac promoter region, the replication origin derived from plasmid pUC18, and all or a part of cDNA of porcine adenylate kinase can be constructed. The vector in which all of cDNA of porcine adenylate kinase is inserted has been proposed by the present inventors as porcine adenylate kinase expression vector pMKAK3 (Japanese Open No 64-51088).

The hirudin expression vector in the present invention can be relatively conveniently constructed by employment of this expression vector.

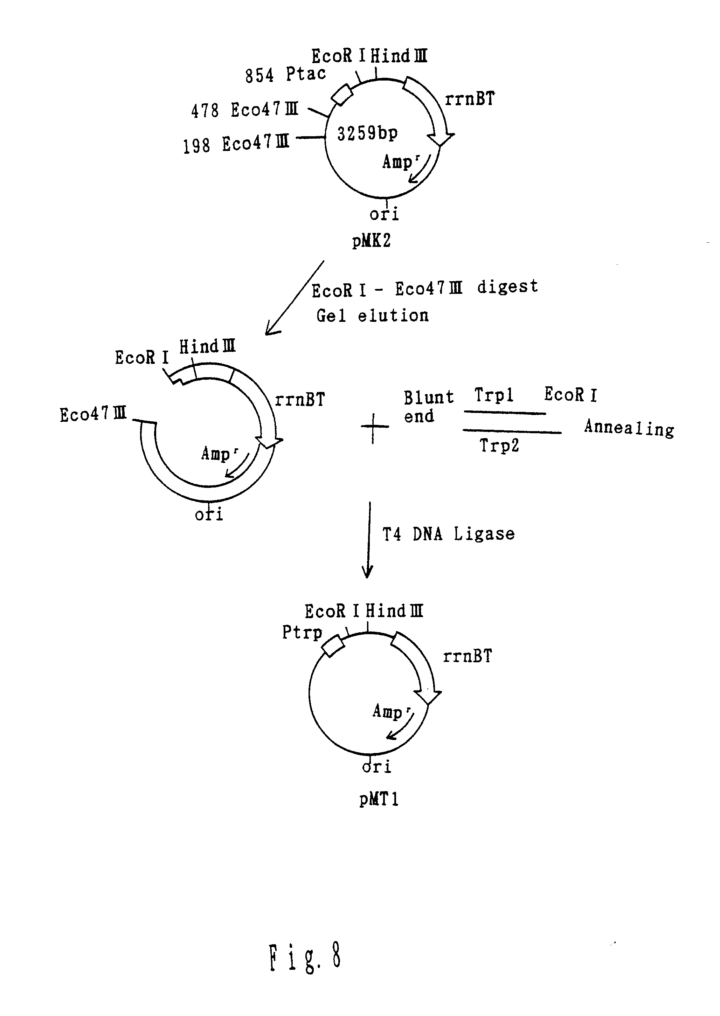

On the other hand, the above mentioned plasmid pMK2 is digested with EcoRI and Eco47III , and larger fragment is isolated. A chemically synthesized fragment of trp promoter is inserted into EcoRI - Eco47III site of the plasmid pMK2 derived fragment to yield plasmid pMT1 as shown in Fig 8.

In the present invention, the above mentioned vector comprising the fragments containing tac or trp promoter region, replication origin derived from plasmid pUC18, and all or a part of porcine adenylate kinase coding sequence may be used. There is no obstacle to employ the vector including other genes than the used plasmid derived one. For example, it is preferentially desirable to use the vector which includes the fragments of rrnB terminator, ampicilin-resistance gene and so on, because its high expression can be carried out and the screening is easy.

Next, the coding sequence of hirudin or its variant can be relatively easily prepared using a DNA synthesizer because hirudin is composed of 65 amino acids, which is relatively small. The codon which is frequently used in Escherichia coli is desirable to design such a gene, especially, the gene which has the nucleotide sequence of HV-1 or HV-3 shown in Fig.1 and Fig.2. This synthesized DNA is inserted into the EcoRI - HindIII site of plasmid pUC18. The plasmids in which the nucleotited sequence of HV-1 and HV-3 inserted are named as pUCHV1 and pUCHV3, respectively.

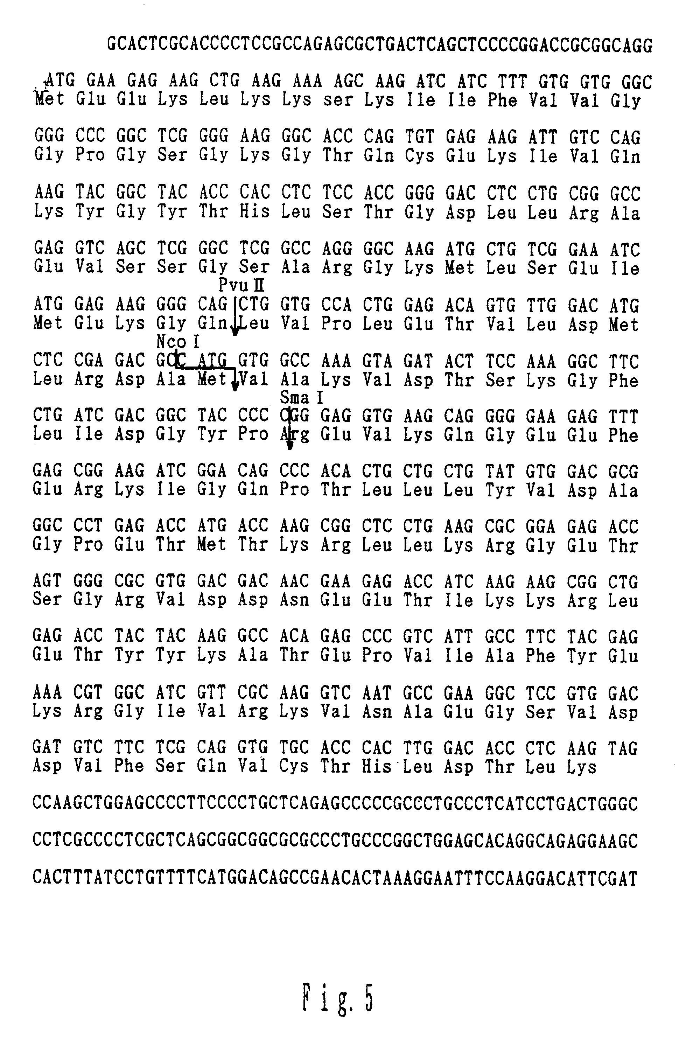

To obtain the expression vector in the present invent ion, plasmid pMKAK3 and plasmid pUCHV1 or pUCHV3 are digested with restriction enzymes. It is desirable to employ Ncol and HindIII or PvuII as restriction enzyme to cleavage the former plasmid pMKAK3. Especially, it is desirable to use restriction enzymes, EcoRI and HindIII in relation to plasmid pUCHV1, and PvuII in relation to plasmid pUCHV3. then these restriction enzymes are used, a DNA of porcine adenylate kinase shown in Fig.5 is cleavaged at the arrow-indicated site with restriction enzymes Nco-I and PvuII .

On the other hand, it is desirable to recover the genes of HV-1 and HV-3 by cleavage of plasmid pUCHV1 with Ncol and HindIII and plasmid pUCHV3 with EcoRI and HindIII , respectively. The restriction sites are illustrated in Fig.1 and Fig.2.

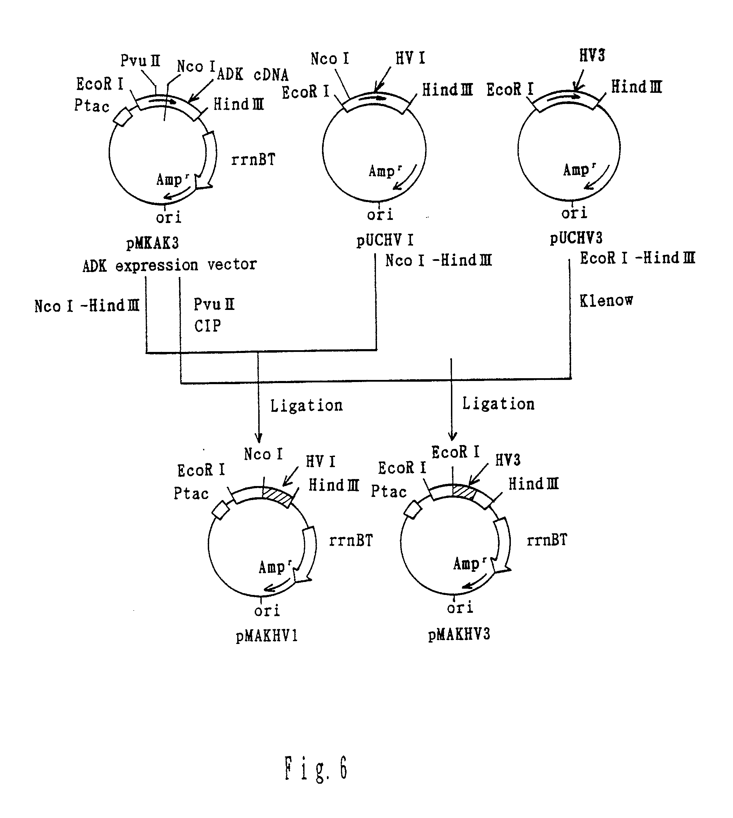

HV-1 gene is isolated by Ncol - HindIII digestion of plasmid pUCHV1 and inserted into Ncol -HindIII site of plasmid pMKAK3, previously obtained, to yield plasmid pMAKHV1 as illustrated in Fig.6 This is done by ligating the two pieces of DNAs using T4 DNA ligase, transforming Escherichia coli with the ligation reaction mixture, followed by selecting a colony harbouring correct plasmid. This plasmid pMAKHV1 comprises DNA fragments containing tac promoter region, replication origin, a part of porcine adenylate kinase coding sequence and hirudin HV-1 coding sequence, which is confirmed by sequencing the plasmid DNA by Sanger method.

Also, HV-3 expression vector can be constructed as follows (Fig.6);

HV-3 gene is isolated by EcoRI -HindIII digestion of plasmid pUCHV3 and following blunt-ending with Klenow. On the other hand, plasmid pMKAK3 is digested with PvuII and treated with alkaline phosphatase to

3

remove 5′-phosphate residue. Escherichia coli is transformed with the ligation mixture of these two DNA pieces, followed by selecting a colony harbouring correct plasmid. Thus obtained plasmid pMAKHV3 comprises DNA fragment containing tac promoter region, replication origin, a part of porcine adenylate kinase coding sequence and hirudin HV-3 coding sequence, which is confirmed by sequencing the plasmid by Sanger method.

Furthermore, the expression vector including trp promoter in the present invention can be constructed as follows;

Plasmid pMT1 is digested with EcoRI and HindIII , and the larger fragment containing trp promoter region and replication origin is isolated.

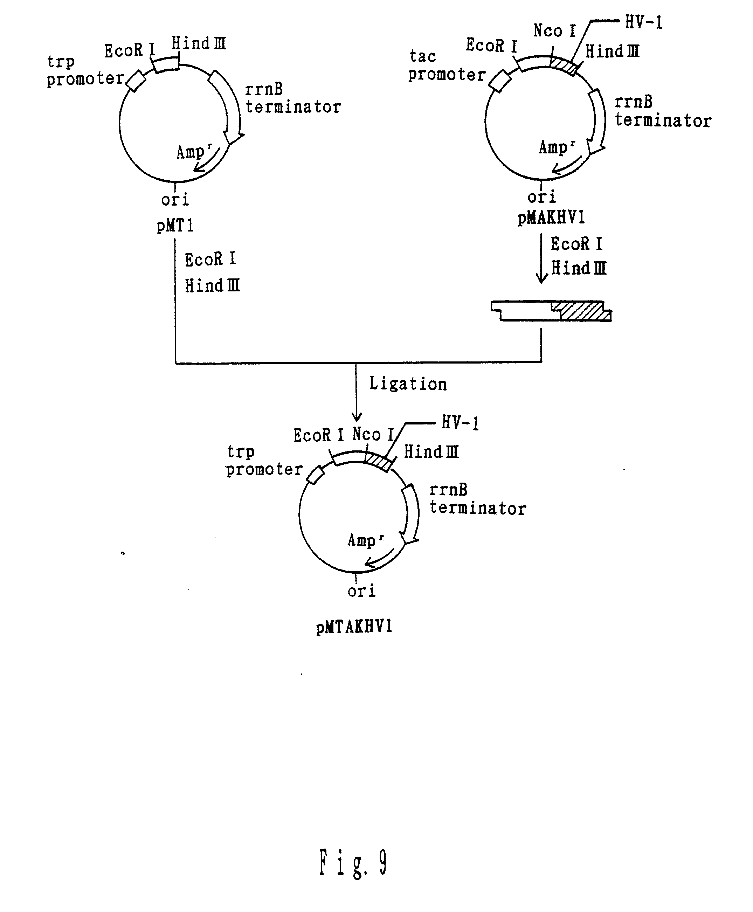

On the other hand, EcoRI and HindIII DNA fragment, of which DNA sequence includes hirudin fusion protein coding region, is isolated from plasmid pMAKHV1. This DNA fragment comprises EcoRI - NcoI fragment encoding partial amino acid sequence of porcine adenylate kinase and NcoI -HindIII fragment encoding amino acid sequence of HV-1 conjugated at NcoI site. The DNA fragment is inserted into EcoRI -Hind III site of the plasmid pMT1 to yield plasmid pMTAKHV1 as shown in Fig.9. This is done by ligating the two pieces of DNAs using T4 DNA ligase, transforming Escherichia coli with the ligation reaction mixture, followed by selecting a colony harbouring correct plasmid.

The plasmid pMTAKHV1 comprises DNA fragments containing trp promoter region, replication origin, a part of porcine adenylate kinase coding sequence, and hirudin HV-1 coding sequence, which is confirmed by sequencing the plasmid by Sanger method.

Also, the DNA of porcine adenylate kinase is isolated by EcoRI -Hind III digestion of plasmid pMKAK3 and inserted EcoRI -HindIII site of plasmid pMT1 to obtain plasmid pMTAK3. And then the plasmid pMTAK3 is digested with PvuII and ligated the above mentioned hirudin HV-3 coding sequence using T4 DNA ligase. Escherichia coli is transformed with the ligation mixture, followed by selecting a colony harbouring correct plasmid. Thus obtained plasmid pMTAKHV3 comprises DNA fragment containing trp promoter region, replication origin of plasmid pUC18, a part of porcine adenylate kinase coding sequence and hirudin HV-3 coding sequence, which is confirmed by sequencing the plasmid by Sanger method.

In the present invention, the transformed microorganism can be obtained by introducing these plasmids pMAKHV1, pMAKHV3, pMTAKHV1 and pMTAKHV3 into Escherichia coli and the screening. Escherichia coli JM105, JM109, and IF 03301 can be used but the microorganisms are not limited to these Escherichia coli 's. The above mentioned fusion protein is produced by culture of transformed microorganisms, and then it is isolated and purified using the techniques of centrifugation, column chromatography and so on following disruption of the microorganisms. In this purification step the fusion protein is treated with cyanogen bromide and so on and devided into fractions of hirudin or its variant and porcine adenylate kinase.

In pMAKHV1, pMAKHV3, pMTAKHV1, pMTAKHV3, porcine adenylate kinase portion in the hirudin fusion protein include three methionine residues at position of 57, 62 and 76 from N terminal in addition to the methionine at fusion site with hirudin (free hirudin is obtained by the cleavage at this site with CNBr treatment) . When the hirudin fusion protein which is generated by the expression of above mentioned vector is cleaved by CNBr treatment in order to obtain hirudin, a small quantitity of hirudin having 5 extra amino acid residues in N-terminal is generated, because it is not cleaved at desired cleavage site of methionine residue, but at the cleavage site of methionine at position 76, and it is very difficult to separate with hirudin. Therefore, to avoid generation of hirudin having extra amino acid residues, vectors, pMTAKHV1M2 and pMAKHV1M3, in which three methionine residues in porcine adenylate kinase are substituted by leucines are made.

The transformed Esherichia coli with these vectors, pMTAKHV1M2 or pMTAKHV1M3 can express the same amount of fusion protein as pMTAHV1 transformed cell, and highly purified HV-1 can be obtained.

The present invention is to provide the expression vector with which hirudin or its variant can be highly expressed and efficiently produced, and thereby to provide the method by which hirudin or its variant can be produced in large quantities and at a low cost using gene technology.

## (5) BRIEF DESCRIPTION OF THE DRAWINGS

Fig.1 and Fig.2 show DNA and amino acid sequences of HV-1 and HV-3 genes, respectively, which are used in the present invention.

Fig.3 and Fig.4 show DNA sequences of tap and trp promoters, respectively.

Fig.5 shows cDNA and amino acid sequences of porcine adenylate kinase which is used in the present invention.

Fig.6 is a scheme of the construction of the expression vectors in the present invention, pMAKHV1 and PMAKHV3.

Fig.7 and Fig.8 are schemes of the construction of plasmids pMK2 and pMT1 respectively, which are used for the preparation of the expression vector in the present invention, pMTAKHV1.

Fig.9 is a scheme of the construction of the expression vector in the present invention, pMTAKHV1 from plasmid pMT1.

Fig. 10 is a scheme of the construction of plasmid pM13AK HV1 from the plasmid pMTAKHV1.

Fig. 11 shows DNA sequences of M57-62L and M76L.

Fig. 12 is a scheme of the construction of the expression vector in the present invention, plasmid pMTAKHV1M3 from plasmid pM13AKHV1M1 and pM13AKHV1M2.

## (6) DETAILED DESCRIPTION OF PREFERED EMBODIMENTS

This invention is shown in detail hereinafter with examples. However, the right of the present invention is not limited in these examples.

EXAMPLES (1)

### (1) Chemical synthesis and purification of DNA fragments of HV-1 and HV-3 genes.

Each DNA fragment was synthesized by the phosphoamidite method using an automatic DNA synthesizer (Applied Biosystems Model 380A). Each DNA fragment with DMT r residue at 5′-end was applied to reverse phase C18 column, and eluted with linear gradient of acetonitril using the acetonitril-0.1M Triethylammonium-acetate (TEA A) buffer. The peak was collected and following its evaporation. 1 ml of 80% acetic acid was added. This was allowed to stand at room temperature for 20 min and then evaporated. The contents were dissolved with water and purified by HPLC using reverse phase C18 column again.

### (2) Combination reaction of the fragments

The genes of HV-1 and HV-3 were synthesized and purified by separating in each 8 fragments as shown in Fig.1 and Fig.2.

In the case of HV-1, 500 pmol of 6 fragments except for 5′-end fragment was reacted with 10 units of T4 polynucleotidekinase and 1.5 μl of 1mM ATP in 50 μl of phosphorylation reaction mixture at 37°C for 1 hr. After heating at 70°C for 3 min the concentration was adjusted to 10 pmol/μl. Each 20 pmol of 8 fragments was mixed, heated for 3 min followed by rapid cooling, heated at 75°C for 10min, and cooled slowly to room temperature for approximately 2 hr to complete the annealing. This was reacted at 16°C overnight in 20 μl of the solution containing T4 DNA ligase (66 mM Tris-Cl, pH7.5, 5mM MgCl₂, 5mM DTT, 1mM ATP). In the case of HV-3, the similar procedure was performed. After the reaction the aimed double-strand DNA was obtained through addition of water to 200 μl, removal of protein with phenol and chloroform, and precipitation with cold ethanol.

### (3) Cloning of HV-1 and HV-3 genes

The plasmid, pUC18 was used as the cloning vector. 10 μg of pUC18 was digested at 37°C for 2hr using 30 units of EcoRI and 30 units of HindIII . This was submitted to agarose gel electrophoresis and the band corresponding to the vector was extracted. Following removal of protein by phenol extraction and precipitation with cold ethanol, it was dissolved in 50 μl of TE buffer (10mM Tris-Cl, pH8.0, 1mM EDTA). To the corresponding amount to 50 ng of this solution 10 μl of the solution containing the double strand DNA obtained as the above (2) (66mMTris-Cl, pH7.5, 5mM MgCl₂, 5mM DTT, 1mM ATP, 300 units of T4 DNA ligase) was added and the reaction was carried out at 16°C overnight. Escherichia coli JM109 was transformed using this reaction mixture and colony of the ampicillin-resistant strain was obtained. Plasmid DNA was prepared from the transformed microorganisms and it was confirmed by the restriction pattern and DNA sequencing that plasmid pUCHV1 in which HV-1 gene was inserted between the restriction sites

EP 0 412 526 A2

of plasmid pUC18 with EcoRI and HindIII and plasmid pUCHV3 in which HV-3 gene was inserted at the same sites were introduced.

(4) Preparation of the expression vector for hirudin fusion protein

① Preparation of the expression vector, pMAKHV1

The DNA fragment including the gene coding for the N-terminal amino acid sequence to methionine at position 80 of porcine adenylate kinase, which was obtained by digestion of 1 $\mu$g of the known expression vector for porcine adenylate kinase, pMKAK3 [Japanese Laid Open No. 64-51088] with 10 units of NcoI and HindIII, was extracted by agarose gel electrophoresis. Also, the DNA fragment coding for 210bp of HV1 gene was obtained by digestion of 10 $\mu$g of plasmid pUCHV1 which included HV-1 gene with 30 units of NcoI and HindIII. This DNA fragment and the DNA fragment which was formed by digestion of the above mentioned plasmid pMKAK3 with NcoI and HindIII were dissolved in the ligase buffer (66mM Tris-Cl, pH7.5, 5mM MgCl$_2$, 5mM DTT, 1mM ATP) and 300 units of T4 DNA ligase was added to this. The reaction was carried out at 16$^\circ$C overnight. Escherichia coli JM109 was transformed using this reaction mixture and the strain which has the aimed recombinant plasmid pMAKHV1 was obtained. This strain is deposited to Fermentation Research Institute as a deposit number FERM BP-2537. The nucleotide sequence of plasmid pMAKHV1 was confirmed by Sanger method .

②Preparation of the expression vector, pMAKHV3

As shown in Fig. 6, after 1 $\mu$g of the above mentioned plasmid pMKAK3 was digested with 10 units of PvuII, 50 $\mu$l of the solution (500mM Tris-Cl, pH9.0, 1mM MgCl$_2$, 1mM ZnCl$_2$) containing 1 unit of alkaline phosphatase BAP was added and the reaction was carried out at 37$^\circ$C for 30 min. This was dissolved in the TE buffer following removal of proteins by phenol extraction and precipitation with cold ethanol. Also, the DNA fragment coding for 210 bp of HV3 gene by digestion of 10 $\mu$g of plasmid pUCHV3 which includes HV-3 gene with 30 units of EcoRI and HindIII was obtained. This DNA fragment was dissolved in 25 $\mu$l of Klenow buffer(10mM Tris-Cl, pH.8.5, 5mM MgCl$_2$) and 2 units of Klenow enzyme was added in the presence of dNTP (dATP, dTTP, dCTP, dGTP) . The reaction was carried out at 37$^\circ$C for 15 min. The DNA fragment of HV-3 gene with the blunt 5$'$-end was obtained by precipitation of this reaction mixture with ethanol.
This DNA fragment and the DNA fragment which is made by digestion the above mentioned plasmid pMKAK3 with PvuII were reacted with T4DNA ligase at 4$^\circ$C overnight. Escherichia coli JM109 was transformed using this reaction mixture and the strain which has the right-directioned recombinant plasmid pMAKHV3 was obtained. This strain was deposited to Fermentation Research Institute as a deposit number FERM BP-2539. The nucleotide sequence of plasmid pMAKHV3 was confirmed by Sanger method.

③ Preparation of the expression vector, pMTAKHV1

(a) Preparation of the plasmid, pMT1

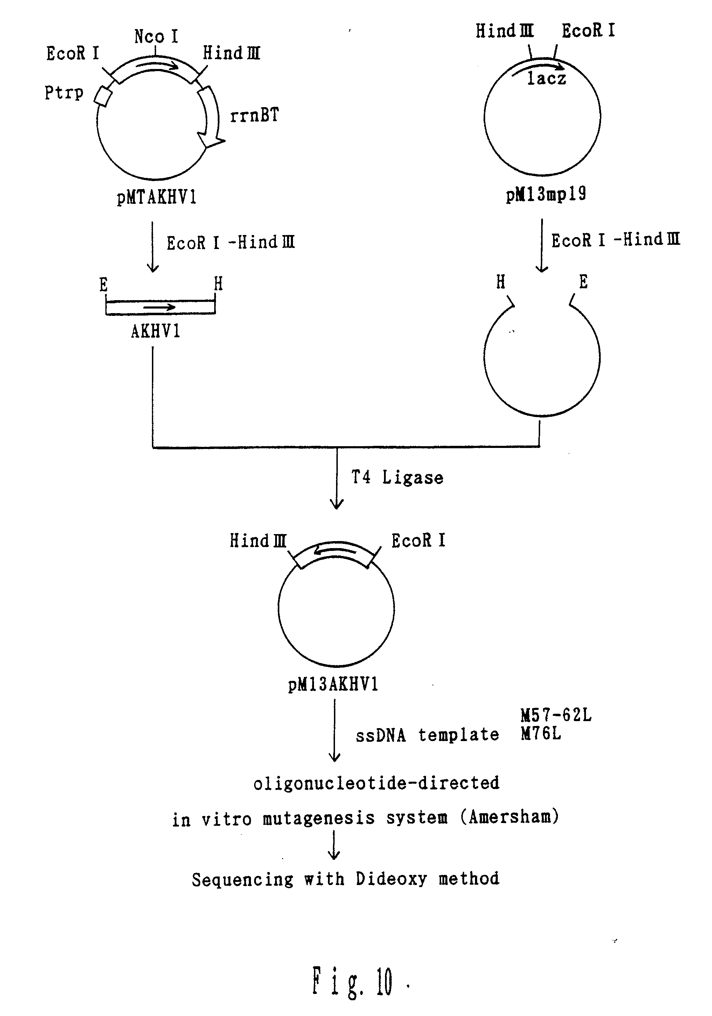

As shown in Fig.7, tac promoter-region which was formed by digestion of plasmid pKK223-3 (Pharmacia), with PvuI and NruI and the fragment including the replication origin and the ampicillin-resistance gene which was formed by digestion of plasmid pUC18 with PvuI and PvuII were combined with T4 DNA ligase. This is introduced into Escherichia coli JM109 and the microorganisms are cultured. The vector including tac promoter and the replication origin of pUC18 was obtained through the screening of the ampicillin-resistance. This plasmid was named as pMK2. As shown in Fig.8, 10$\mu$g of this plasmid was digested with 30 units of EcoRI and ECO47III and the DNA fragment including tac promoter was removed. The DNA fragment including the replication origin was iosolated by agarose gel electrophoresis. On the other hand, the nucleotide sequence of trp promotor shown in Fig.4 was synthesized by its division of 2 parts of TRP-1 and TRP-2 using a DNA synthesizer. This was purified by HPLC using reverse phase C18 column and the double-strand DNA was obtained by annealing of each 100 pmol of this. This DNA fragment

6

and the DNA fragment which is made by digestion the above mentioned pMK2 with EcoRI and Eco47III reacted with T4 DNA ligase at 16° C overnight. Escherichia coli JM109 was transformed using this reaction mixture and the vector including trp promoter and the replication origin of pMK2 was obtained. The nucleotide sequence of this plasmid was confirmed by Sanger method and this plasmid was named as pMT1.

(b) Preparation of the plasmid, pMTAKHV1

As shown in Fig.9, 1 μg of the above mentioned plasmid pMT1 was digested with 10 units of EcoRI and HindIII and the band corresponding to the vector was isolated by agarose gel electrophoresis. On the other hand, 10 μg of pMAKHV1 was digested with 30 unitsof EcoR I and HindIII and the DNA fragment coding for the fusion protein gene was isolated from the gel. This DNA fragment and the DNA fragment which is made by digestion the above mentioned pMT1 with EcoRI and ECO47III reacted with T4 DNA ligase at 16° C overnight. Escherichia coli JM109 was transformed using this reaction mixture and the strain which has the aimed recombinant plasmid pMTAKHV1 was obtained. This strain was deposited to Fermentation Research Institute as a deposit number FERM BP-2538. The nucleotide sequence of plasmid pMTAKHV1 was confirmed by Sanger method .

(c) Preparation of the plasmid, pMTAKHV3

10 μg of the pMKAK3 was digested with 30 units of EcoRI and HindIII and the DNA fragment of porcine adenylate kinase gene was isolated by agarose gel electrophoresis. This DNA fragment and the DNA fragment which is made by digestion the above mentioned plasmid pMT1 with EcoRI and HindIII reacted with T4 DNA ligase at 16° C overnight. Escherichia coli JM109 was transformed using this reaction mixture and the strain which has the aimed recombinant plasmid pMTAK3 was obtained. After 1 μg of plasmid pMTAK3 was digested with 10 units of PvuII , 50 μl of the solution (500mM Tris-Cl, pH9.0, 1mM MgCl$_2$, 1mM ZnCl$_2$) containing 1 unit of alkaline phosphatase BAP was added and the reaction was carried out at 37° C for 30 min. This was dissolved in the TE buffer following removal of proteins by phenol extraction and precipitation with cold ethanol. This DNA fragment and the DNA fragment of HV-3 gene which was blunt-ended with Klenow enzyme were reacted with T4 DNA ligase at 4° C overnight. Escherichia coli JM109 was transformed using this reaction mixture and the strain which has the aimed recombinant plasmid pMTAKHV3 was obtained. This strain was deposited to Fermentation Research Institute as a deposit number FERM BP-2540. The nucleotide sequence of plasmid pMTAKHV3 was confirmed by Sanger method.

(5) Expression of hirudin

Escherichia coli JM109 was transformed by the above mentioned plasmid pMAKHV1, pMAKHV3, pMTAKHV1 and pMTAKHV3 was cultured in 2XTY medium containing 50 μg/ml ampicillin (16 g/l Bacto trypton, 10 g/l Bacto-yeast extract, 5 g/l NaCl, pH7.2). Also, Escherichia coli JM109 which was transformed by pMTAKHV1 and pMTAKHV3 was cultured in M9 medium containing 50 μg/ml ampicillin (5 g/l glucose, 5g/l casamino acid, 6g/l Na$_2$HPO$_4$, 3g/lKH$_2$PO$_4$, 0.5 g/lNaCl, 1 g/l NH$_4$Cl, 10mg/l thiamine hydrochloride, 1mM MgSO$_4$, 100 μM CaCl$_2$, 50mg/l tryptophan, pH7.2) at 37° C overnight. After the culture 1 μl was collected and suspended in Remli's sample buffer. After it was dissolved by heating it was applied to SDS-polyacrylamide gel electrophoresis. The polypeptide bands at molecular weight 17,000 and 14,000 in the cases of the fusion proteins of HV-1 and HV-3, respectively, were seen following dyeing the gel with Coomasie brilliant blue and decoloring. The strains JM105 and IFO 3301 were used and the expression efficiency in various hosts was studied. These results are shown in Table 1. These results indicate that every strains showed high expression, especially, Escherichia coli JM109 and IFO 3301 in which pMTAKHV1 including trp promoter were introduced showed high expression.

Table 1

| Vector | Host | Culture medium | Expression | | |
|---|---|---|---|---|---|
| | | | expression% against all proteins | optical density at A660 | hirudin quantity produced in medium (mg%)* |
| ①pMAKHV1 | JM109 | 2XTY | 13.8 | 2.57 | 27.8 |
| ②pMAKHV1 | JM105 | 2XTY | 7.9 | 2.34 | 14.4 |
| ③ pMTAKHV1 | JM109 | M9 | 17.9 | 1.27 | 17.8 |
| ④ pMTAKHV1 | JM105 | M9 | 9.0 | 2.24 | 15.8 |
| ⑤ pMTAKHV1 | IFO 3301 | M9 | 9.1 | 1.99 | 14.2 |
| ⑥ pMTAKHV1 | JM109 | 2XTY | 8.1 | 2.57 | 16.4 |
| ⑦ pMTAKHV1 | JM105 | 2XTY | 11.5 | 2.47 | 22.4 |
| ⑧ pMTAKHV1 | IFO3301 | 2XTY | 12.8 | 2.94 | 29.4 |

* calculated from 0.175g.l of protein content at A660 = 1.0

(6) Purification of hirudin from culture medium

Transformed microorganisms were collected by low speed centrifugation from 2 l of the above mentioned culture medium. The obtained pellets were suspended in 300ml of $H_2O$, adjusted to pH 4 with $H_2SO_4$, disrupted the cells and centrifuged. The obtained pellets were washed with 200 ml of isopropanol and acetone respectively, then dried and dissolved in 200 ml of 70% formic acid (less than 20g/l ,protein). CNBr was added in final concentration of 1% and stand out at room temperature for overnight. After the CNBr treatment, they were concentrated and dried at 40°C in the reduced pressure, then dissolved in 400 ml of buffer (0.1M Tris-Cl (pH8.7), 0.1M $(NH_4)_2SO_4$ , 0.1mM TritonX-100) . PH of the mixture was adjusted to 8.7 with NaOH. The mixture was added in concentration of 6mM of L-cystein and stand out at room temperature for overnight. After the addition of ammonium sulphate to final concentration of 2 M, immediately centrifuged. The obtained supernatant was loaded on Butyl-toyo pearl (TOSO Co. LTD) which is equilibrated with 50 mM sodium phosphate buffer pH7.0 containing 2M ammonium sulphate, washed with same buffer and then eluted with 50 mM sodium phosphate buffer pH7.0 containing 1.5 M ammonium sulphate. The eluted fractions were treated with either gel filtration or dialysis in order to remove amonium salt. After desalting, loaded on Q-Sepharose or mono-Q Sepharose ( both Pharmacia) which is equilibrated with 50mM sodium phosphate buffer pH7.5 then eluted with 5mM sodium phosphate containing 100mM NaCl. Purified hirudin was obtained from eluted fraction.

EXAMPLES (2)

(1) Construction of fragment substituted methionine-codon by leucin-codon

10 μg of pMTAKHV1 obtained with EXAMPLES (1) was digested with 30 units of EcoRI and HindIII. The part of adenylate kinase-hirudin was isolated by agarose gel electrophoresis.

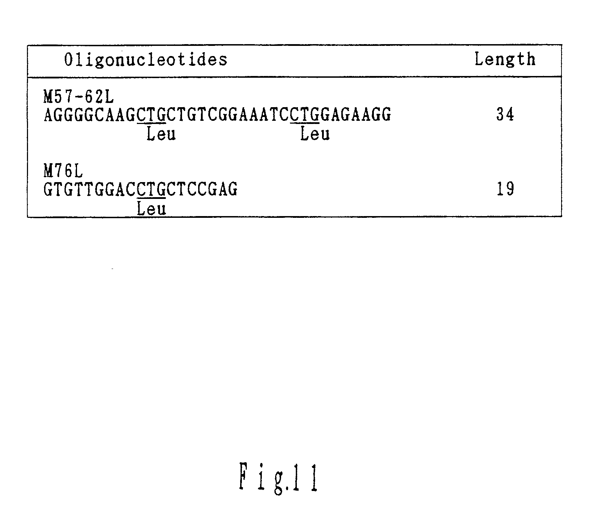

On the other hand, 1 μg of phage vector, pM13mp19 was digested also with 10 units of EcoRI and HindIII and large fragment was collected from agarose gel as shown in Fig.10. Both of these large fragment and DNA fragment coding for above mentioned adenylate kinase-hirudin HV-1 were reacted with T4 ligase at 16°C for overnight. Site-directed mutagenesis was performed by oligonucleotide directed in vitro mutagenesis system with above mentioned pM13 vector, M13AKHV1 coding for adenylate kinase-hirudin HV-1, and single strand DNA templates, M57-62 and M76L which were synthesized by DNA synthesizer, as shown in Fig.11.

Thus, pM13AKHV1M1 with which both site of methionine codon(ATG) of 57 and 62 are substituted by codon(CTG) of leucine was obtained, and pM13AKHV1M2 with which a site of methionine codon of 76 is subsituted by leucine (CTG) was also obtained. The DNA sequences were confirmed by dideoxy method.

(2) Construction of expression vectors pMTAKHV1M1, pMTAKHV1M2, pMTAKHV1M3

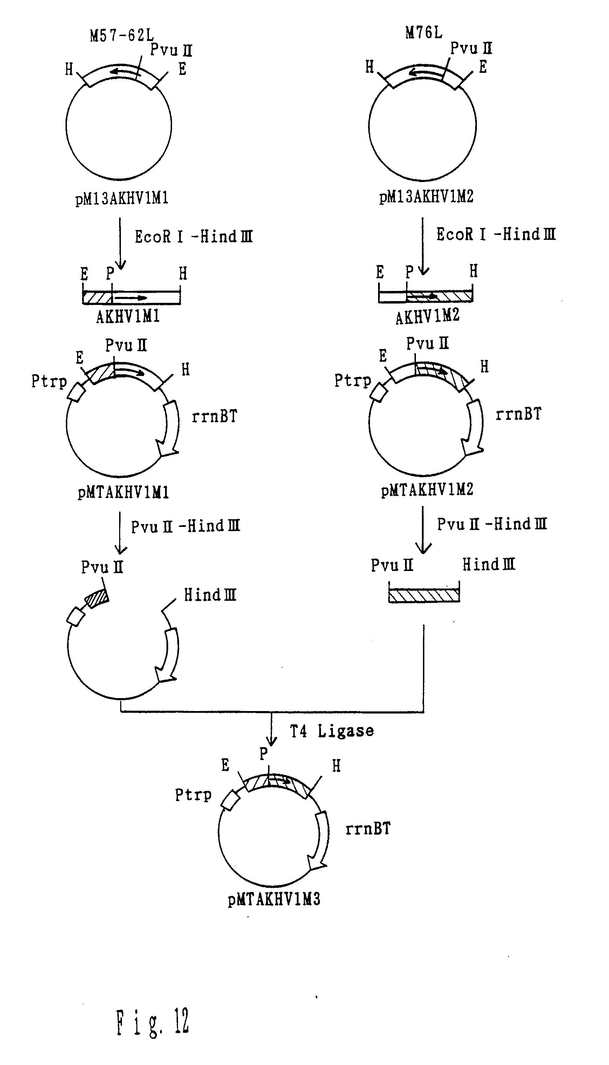

Next, as shown in Fig.12, the adenylate kinase-hirudin parts which were formed by digestion of pM13AKHV1M1 or pM13AKHV1M2 with EcoRI and HindIII and pMTAKHV1 with which the adenylate kinase-hirudin part was removed by digestion were combined to construct pMTAKHV1M1 or pMTAKHV1M2 respectively.

Then, the large fragment with which pMTAKHV1M1 was digested with PvuII and HindIII and the small fragment with which pMTAKHV1M2 was digested with PvuII and HindIII were jointed to construct pMTAKHV1M3 with which all of three methionine codon were substituted by leucine codon. The DNA sequence was confirmed.

(3) Expression of hirudin

Escherichia coli JM 109 were transformed with pMTAKHV1M2 or pMTAKHV1M3, expressed hirudin HV-1 as descrived in EXAMPLE(1). Those strains are deposited to Fermentation Research Institute as deposit number FERM BP-2988 and FERM BP-2989, resp. The expression of hirudin HV-1 with pMTAKHV1M2 or pMTAKHV1M3 showed equal degree as compared pMTAKHV1 respectively. It was confirmed that expression of hirudin HV-1 with pMTAKHV1M2 or pMTAKHV1M3 was differed from expression of pMTAKHV1 in no-generation of hirudin having 5 extra amino acid residues of N-terminal.

**Claims**

(1) An expression vector for hirudin which comprises of DNA fragments of tac or a trp promoter region, replication origin of plasmid pUC18, all or a part of porcine adenylate kinase coding sequence, and hirudin or its variant coding sequence, and which is characterized to express a fused protein of hirudin or its variant with all or a part of porcine adenylate kinase.

(2) A hirudin expression vector which is named by pMKAHV1 according to claim (1).

(3) A hirudin expression vector which is named by pMAKHV3 according to claim (1).

(4) A hirudin expression vector which is named by pMTAKHV1 according to claim (1)

(5) A hirudin expression vector which is named by pMTAKHV3 according to claim (1).

(6) A hirudin expression vector in which all or a part of methionine residues in porcine adenylate kinase are substituted by other amino acids than methionine according to claim (1).

(7) A hirudin expression vector which is named by pMTAKHV1M2 according to claim (6).

(8) A hirudin expression vector which is named by pMTAKHV1M3 according to claim (6).

(9) A transformed microorganism which is made by the transduction of the vector according to any one of claim (1) to (8) into Escherichia coli .

(10) A transformed microorganism which is made by introducing plasmid, pMAKHV1 to Escherichia coli JM109 (FERM BP-2537) according to claim (9).

(11) A transformed microorganism which is made by introducing plasmid, pMAKHV3 to Escherichia coli JM 109 (FERM BP-2539) according to claim (9).

(12) A transformed microorganism which is made by introducing plasmid, pMTAKHV1 to Escherichia coli JM109 (FERM BP-2538) according to claim (9).

(13) A transformed microorganism which is made by introducing plasmid, pMTAKHV3 to Escherichia coli JM109 (FERM BP-2540) according to claim (9).

(14) A transformed microorganism which is made by introducing plasmid, pMTAKHV1M2 to Escherichia coli JM109 (FERM BP-2988) according to claim (9).

(15) A transformed microorganism which is made by introducing plasmid, pMTAKHV1M3 to Escherichia coli JM109 FERM BP-2989) according to claim (9).

(16) Method for a production of hirudin, which is characterized in that the transformed microorganism according to any one of claim (9) to (15) is cultured, the hirudin fusion protein is expressed, and hirudin or its variant is recovered by cleavage of the fusion protein following its extraction.

(17) A hirudin fusion protein which is formed by the combination of all or a part of porcine adenylate kinase and hirudin or its variant.

(18) A hirudin fusion protein which is formed by the combination of all or a part of porcine adenylate kinase in which all or a part of methionine residues adenylate kinase are substituted by other amino acids than methionine, and hirudin or its variant according to claim (17).

(19) DNA sequence indicated by the following general formula.

AGGGGCAAGCTGCTGTCGGAAATCCTGGAGAAGG

(20) DNA sequence indicated by the following general formula.

GTGTTGGACCTGCTCCGAG

EP 0 412 526 A2

```
                    5                       10                      15
      MET Val Val Tyr Thr Asp Cys Thr Glu Ser Gly Gln Asn Leu Cys leu
     AAT TCC ATG GTT GTA TAC ACT GAT TGT ACT GAA TCT GGC CAG AAC CTG TGT CTG (HV-1-101,54mer)
      GG TAC CAA CAT ATG TGA CTA ACA TGA CTT AGA CCG GTC TTG GAC ACA GAC (HV-1-201,60mer)
     EcoR I  Nco I     Acc I                              Bal I


                   20                      25                      30
      Cys Glu Gly Ser Asn Val Cys Gly Gln Gly Asn Lys Cys Ile Leu Gly Ser Asp
     TGT GAA GGA TCC AAC GTT TGT GGT CAG GGT AAC AAA TGT ATC CTC GGG TCT GAT (HV-1-102,54mer)
     ACA CTT CCT AGG TTG CAA ACA CCA GTC CCA TTG TTT ACA TAG GAG CCC AGA CTA (HV-1-202,54mer)
             BamH I                                            Ava I


        35                      40                      45                      50
      Gly Glu Lys Asn Gln Cys Val Thr Gly Glu Gly Thr Pro Lys Pro Gln Ser His
     GGT GAA AAG AAC CAG TGT GTT ACT GGT GAA GGT ACC CCG AAA CCG CAG TCT CAT (HV-1-103,54mer)
     CCA CTT TTC TTG GTC ACA CAA TGA CCA CTT CCA TGG GGC TTT GGC GTC AGA GTA (HV-1-203,54mer)
                                              Kpn I


        55                      60                      65
      Asn Asp Gly Asp Phe Glu Glu Ile Pro Glu Glu Tyr Leu Gln
     AAC GAT GGT GAT TTC GAA GAA ATC CCG GAA GAA TAC CTG CAG TAG TAA A     (HV-1-104,49mer)
     TTG CTA CCA CTA AAG CTT CTT TAG GGC CTT CTT ATG GAC GTC ATC ATT TTC GA  (HV-1-204,43mer)
                                              Pst I              Hind III
```

# Fig. 1

```
                          5                      10                      15
           MET Ile Thr Tyr Thr Asp Cys Thr Glu Ser Gly Gln Asn Leu Cys Leu
AAT TCC ATG ATT ACG TAT ACT GAT TGT ACT GAA TCT GGC CAG AAC CTG TGT CTG    (HV-3-101,54mer)
    GG TAC TAA TGC ATA TGA CTA ACA TGA CTT AGA CCG GTC TTG GAC ACA GAC    (HV-3-201,60mer)
    EcoR I                ACC I                     BaL I


                         20                      25                      30
       Cys Glu Gly Ser Asn Val Cys Gly Lys Gly Asn Lys Cys Ile Leu Gly Ser Gln
TGT GAA GGA TCC AAC GTT TGT GGT AAA GGT AAC AAA TGT ATC CTC GGG TCT CAG    (HV-3-102,54mer)
ACA CTT CCT AGG TTG CAA ACA CCA TTT CCA TTG TTT ACA TAG GAG CCC AGA GTC    (HV-3-202,54mer)
           BamH I                                              Ava I


       35                      40                      45                      50
   Gly Lys Asp Asn Gln Cys Val Thr Gly Glu Gly Thr Pro Lys Pro Gln Ser His
GGT AAA GAT AAC CAG TGT GTT ACT GGT GAA GGT ACC CCG AAA CCG CAG TCT CAT    (HV-3-103,54mer)
CCA TTT CTA TTG GTC ACA CAA TGA CCA CTT CCA TGG GGC TTT GGC GTC AGA GTA    (HV-3-203,54mer)
                                          Kpn I


       55                      60                      65
   Asn Gln Gly Asp Phe Glu Pro Ile Pro Glu Asp Ala Tyr Asp Glu
AAC CAG GGT GAT TTC GAA CCG ATC CCG GAA GAC GCG TAC GAT GAA TAG TAA A      (HV-3-104,52mer)
TTG GTC CCA CTA AAG CTT GGC TAG GGC CTT CTG CGC ATG CTA CTT ATC ATT TTC GA (HV-3-204,46mer)
                                      Mlu I                     Hind III
```

# Fig. 2

EP 0 412 526 A2

```
        -35                                    -10
5′ -TG T TGA CA A TTA ATC ATC GGC TCG TAT AAT G TG TGG AAT TGT GAG
    ACA ACT GTT AAT TAG TAG CCG AGC ATA TTA CAC ACC TTA ACA CTC

                            S/D       EcoR I
   CGG ATA ACA ATT TCA CAC AGG A AA CAG AAT TC - 3′
   GCC TAT TGT TAA AGT GTG TCC TTT GTC TTA A
```

# Fig. 3

```
        -35                                            -10
5′ -GGG TG T TGA CA A TTA ATC ATC GAA CTA G TT AAC T AG TAC GCA AGT
    CCC ACA ACT GTT AAT TAG TAG CTT GAT CAA TTG ATC ATG CGT TCA

                    S/D       EcoR I
   TCA CGT AAA AAG G GT AG -3′              ( TRP1 )
   AGT GCA TTT TTC CCA TCT TA A             ( TRP2 )
```

# Fig. 4

GCACTCGCACCCCTCCGCCAGAGCGCTGACTCAGCTCCCCGGACCGCGGCAGG

```
 ATG GAA GAG AAG CTG AAG AAA AGC AAG ATC ATC TTT GTG GTG GGC
 Met Glu Glu Lys Leu Lys Lys ser Lys Ile Ile Phe Val Val Gly

GGG CCC GGC TCG GGG AAG GGC ACC CAG TGT GAG AAG ATT GTC CAG
Gly Pro Gly Ser Gly Lys Gly Thr Gln Cys Glu Lys Ile Val Gln

AAG TAC GGC TAC ACC CAC CTC TCC ACC GGG GAC CTC CTG CGG GCC
Lys Tyr Gly Tyr Thr His Leu Ser Thr Gly Asp Leu Leu Arg Ala

GAG GTC AGC TCG GGC TCG GCC AGG GGC AAG ATG CTG TCG GAA ATC
Glu Val Ser Ser Gly Ser Ala Arg Gly Lys Met Leu Ser Glu Ile
                     Pvu II
ATG GAG AAG GGG CAG|CTG GTG CCA CTG GAG ACA GTG TTG GAC ATG
Met Glu Lys Gly Gln↓Leu Val Pro Leu Glu Thr Val Leu Asp Met
                Nco I
CTC CGA GAC GCC ATG GTG GCC AAA GTA GAT ACT TCC AAA GGC TTC
Leu Arg Asp Ala Met↓Val Ala Lys Val Asp Thr Ser Lys Gly Phe
                     Sma I
CTG ATC GAC GGC TAC CCC CGG GAG GTG AAG CAG GGG GAA GAG TTT
Leu Ile Asp Gly Tyr Pro Arg Glu Val Lys Gln Gly Glu Glu Phe
                        ↓
GAG CGG AAG ATC GGA CAG CCC ACA CTG CTG CTG TAT GTG GAC GCG
Glu Arg Lys Ile Gly Gln Pro Thr Leu Leu Leu Tyr Val Asp Ala

GGC CCT GAG ACC ATG ACC AAG CGG CTC CTG AAG CGC GGA GAG ACC
Gly Pro Glu Thr Met Thr Lys Arg Leu Leu Lys Arg Gly Glu Thr

AGT GGG CGC GTG GAC GAC AAC GAA GAG ACC ATC AAG AAG CGG CTG
Ser Gly Arg Val Asp Asp Asn Glu Glu Thr Ile Lys Lys Arg Leu

GAG ACC TAC TAC AAG GCC ACA GAG CCC GTC ATT GCC TTC TAC GAG
Glu Thr Tyr Tyr Lys Ala Thr Glu Pro Val Ile Ala Phe Tyr Glu

AAA CGT GGC ATC GTT CGC AAG GTC AAT GCC GAA GGC TCC GTG GAC
Lys Arg Gly Ile Val Arg Lys Val Asn Ala Glu Gly Ser Val Asp

GAT GTC TTC TCG CAG GTG TGC ACC CAC TTG GAC ACC CTC AAG TAG
Asp Val Phe Ser Gln Val Cys Thr His Leu Asp Thr Leu Lys
```

CCAAGCTGGAGCCCCTTCCCCTGCTCAGAGCCCCCGCCCTGCCCTCATCCTGACTGGGC

CCTCGCCCCTCGCTCAGCGGCGGCGCGCCCTGCCCGGCTGGAGCACAGGCAGAGGAAGC

CACTTTATCCTGTTTTCATGGACAGCCGAACACTAAAGGAATTTCCAAGGACATTCGAT


# Fig. 5

Fig. 6

Fig. 7

Fig. 8

17

Fig. 9

EcoR I    Nco I    Hind Ⅲ

Ptrp

rrnBT

pMTAKHV1

Hind Ⅲ    EcoR I

lacz

pM13mp19

EcoR I -Hind Ⅲ

EcoR I -Hind Ⅲ

E      H

AKHV1

H      E

T4 Ligase

Hind Ⅲ      EcoR I

pM13AKHV1

ssDNA template    M57-62L

M76L

oligonucleotide-directed

in vitro mutagenesis system (Amersham)

Sequencing with Dideoxy method

F i g. 10 .

| Oligonucleotides | Length |
|---|---|
| M57-62L<br>AGGGGCAAG<u>CTG</u>CTGTCGGAAATC<u>CTG</u>GAGAAGG<br>      Leu               Leu | 34 |
| M76L<br>GTGTTGGAC<u>CTG</u>CTCCGAG<br>      Leu | 19 |

Fig.11

Fig. 12